(19) Europäisches Patentamt
European Patent Office
Office européen des brevets

(11) Veröffentlichungsnummer: **0 012 444**
**B1**

(12) # EUROPÄISCHE PATENTSCHRIFT

(45) Veröffentlichungstag der Patentschrift: **25.07.84**

(21) Anmeldenummer: **79105191.5**

(22) Anmeldetag: **14.12.79**

(51) Int. Cl.³: **G 01 N 33/50,**
A 61 B 10/00

(54) **Diagnostizierverfahren, insbesondere zur Früherkennung von malignen Tumoren und/oder viralen Erkrankungen und Mittel zu seiner Durchführung.**

(30) Priorität: **18.12.78 AT 9005/78**
**15.06.79 AT 4242/79**
**25.10.79 AT 6930/79**

(43) Veröffentlichungstag der Anmeldung:
**25.06.80 Patentblatt 80/13**

(45) Bekanntmachung des Hinweises auf die Patenterteilung:
**25.07.84 Patentblatt 84/30**

(84) Benannte Vertragsstaaten:
**AT BE CH DE FR GB IT LU NL SE**

(56) Entgegenhaltungen:
**Kokolis, N. Ziegler; J. Cancer Biochem. Biophys. 1977, Vol. 2, pp. 79-85**

(73) Patentinhaber: **Wachter, Helmut, Univ.-Prof. Dr.**
**Reithmannstrasse 18**
**A-6020 Innsbruck (AT)**

(72) Erfinder: **Wachter, Helmut, Prof. Dr.**
**Reithmannstrasse 18**
**A-6020 Innsbruck (AT)**
Erfinder: **Hausen, Arnulf Dr.**
**Kochholzweg 166**
**A-6020 Innsbruck-Lans (AT)**
Erfinder: **Grossmayr, Nikolaus Dr.**
**Sistrans Nr. 65**
**A-6020 Innsbruck (AT)**

(74) Vertreter: **Torggler, Paul, Dr. et al,**
**Patentanwälte Dr. Paul Torggler DDr. Engelbert Hofinger Wilhelm-Greil-Strasse 16**
**A-6020 Innsbruck (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 012 444 B1

**Beschreibung**

Die Erfindung bezieht sich auf ein Diagnostizierverfahren, insbesondere zur Früherkennung von malignen Tumoren und/oder viralen Erkrankungen und Mittel zu seiner Durchführung im menschlichen oder tierischen Organismus, wobei eine dem Organismus entnommene Körperflüssigkeit auf den Gehalt bestimmter Pteridine untersucht wird, sowie diagnostische Mittel zur Durchführung des Verfahrens.

Die Diagnose maligner Tumoren erfolgt heute fast ausschließlich aufgrund von klinischen, radiologischen, endoskopischen, histologischen und zytologischen Untersuchungen. Klinisch chemische oder biochemische Befunde, die für einen malignen Tumor eine sichere Voraussage zulassen, existieren bis heute noch nicht. Speziell bei endokrin wirksamen Geschwülsten, die vermehrt Hormone produzieren, kann zwar die quantitative Bestimmung dieser Hormone im Serum oder im Urin wichtige diagnostische Hinweise liefern. Die erhöhten Hormonwerte lassen jedoch nicht sicher auf maligne Neoplasien schließen, sondern können auch bei anderen Erkrankungen auftreten.

In neuerer Zeit hat auch die Bestimmung einiger tumorassoziierter Antigene, z.B. des carcinoembryonalen Antigens (CEA) oder des $\alpha$-Fetoproteins eine gewisse Bedeutung erlangt. Da diese Antigene einerseits je nach Tumor im besten Falle nur bei 30—90% der Tumorpatienten erhöht sind, andererseits auch bei Patienten mit anderen Erkrankungen erhöhte Werte gefunden werden, bleibt der praktische Wert dieser Untersuchungen sehr begrenzt.

Erfolgversprechender, obwohl bisher in der Praxis noch nicht verwendet, scheint eine Tumordiagnose aufgrund der Bestimmung des Gehaltes bestimmter Pteridine in Körperflüssigkeiten. Kokolis und Ziegler (Kokolis N. und Ziegler I., Cancer Biochem. Biophys. 1977, Vol. 2, pp. 79—85) fanden im Serum von Tumor-Patienten erhöhte Werte von Tetrahydrobiopterin. Biopterin bzw. Tetrahydrobiopterin wurden offenbar auch deshalb mit dem Wachstum von Krebszellen in Verbindung gebracht, weil es bei fundamentalen Reaktionen im Stoffwechsel als Cofaktor beteiligt ist und weil es überdies einen Wuchsstoff bei bestimmten Mikroorganismen (Crithidia Fasciculata) darstellt. Untersuchungen von Halpern et al. (Cancer Research 38, 2378—2384, 1978) ergaben in Gewebskulturen von malignen Zellen erhöhte Mengen von 6-Hydroxymethylpterin nach vorheriger Gabe von Folsäure. Diese ersten Untersuchungen bringen die Pteridinausscheidung mit dem Folsäurestoffwechsel in Verbindung, wobei auch die Folsäure selbst ein Wuchsstoff für bestimmte Mikroorganismen ist.

Die klinische Laboratoriumsdiagnostik der Viruskrankheiten beruht derzeit im wesentlichen auf drei Verfahrensprinzipien:

1) Isolierung, Züchtung und Identifizierung des Virus vom Kranken (Dauer der Untersuchung ca. 6 Wochen);

2) Nachweis von virusspezifischen Antikörpern im Serum oder Liquor des Kranken (Dauer der Untersuchung ca. 3 Wochen);

3) Züchtung und Identifizierung von Bakterien des Kranken, bei negativem Ergebnis Ausschluß der bakteriellen Erkrankung und Annahme einer viralen Erkrankung (indirekter Nachweis — Dauer der Untersuchung 2 bis 3 Tage, Wiederholungsuntersuchungen erforderlich).

Die unter Punkt 1 genannten Methoden, die auch als "große Virusdiagnostik" bezeichnet werden, sind kostspielig und mühevoll. Sie sind auch für die Klinik nicht immer befriedigend; dies gilt z.B. dann, wenn Erkrankungen der oberen Luftwege oder meningoencephalitische Syndrome aufgeklärt werden sollen. Die Isolierung und Identifizierung bedeuten in diesen Situationen für den Einzelfall keine diagnostische Hilfe, weil ihre Resultate zu spät ablesbar sind.

Die unter Punkt 2 genannten Methoden sind einfacher auszuführen und für den Klinikbetrieb von größerer Bedeutung.

a) Der Neutralisationstest

b) Der Hämagglutinations-Hemmungstest (Hirst-Test)

c) Die Komplementbindungsreaktion (KBR).

Für all diese Untersuchungen ist es notwendig, daß sie gleichzeitig an zwei Serumproben des gleichen Kranken quantitativ ausgeführt werden. Die erste Serumprobe soll sogleich nach dem Erscheinen der klinischen Symptome entnommen werden, die zweite Serumprobe nach etwa 14 Tagen. Die Serumproben werden sofort nach Entnahme eingeschickt und im Laboratorium eingefroren. Die Untersuchung des Serumpaares erfolgt stets am gleichen Tag. Auch bei dieser "kleinen Virusdiagnostik" ergibt sich ein wesentlicher Zeitfaktor und erheblicher diagnostischer und kostspieliger Aufwand.

Beim dritten Verfahren handelt es sich um ein Ausschlußverfahren mit allen diesen Verfahren anhaftenden Mängeln. Die Viruserkrankung selbst wird dabei nicht direkt nachgewiesen.

Aufgabe der Erfindung ist es, unabhängig vom Folsäurestoffwechsel die Ausscheidung bestimmter Pteridine, ausgenommen Biopterin, in Körperflüssigkeiten für ein in der medizinisch-technischen Praxis brauchbares Diagnostizierverfahren, insbesondere zur Früherkennung von malignen Tumoren und/oder Viruserkrankungen einzusetzen.

Dies wird erfindungsgemäß dadurch erreicht, daß in der Körperflüssigkeit 7,8-Dihydropteridin bzw. seine Oxidationsprodukte oder seine Reduktionsprodukte oder deren substituierten Produkte, ausgenommen Biopterin und seine Reduktionsprodukte, qualitativ nachgewiesen und/oder halbquantitativ bzw. quantitativ bestimmt werden.

Das 7,8-Dihydropteridin hat folgende Formel:

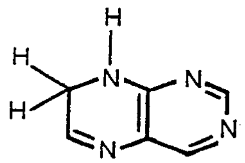

Beispiele für Dihydropteridine sind:

das 7,8-Dihydropterin

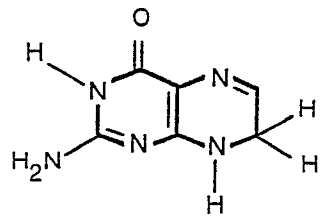

das 7,8-Dihydrolumazin

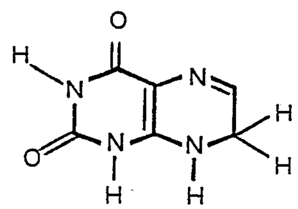

das 7,8-Dihydroxanthopterin

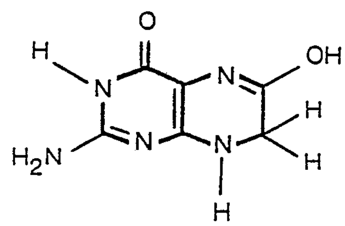

die 7,8-Dihydro-7-xanthopterincarbonsäure

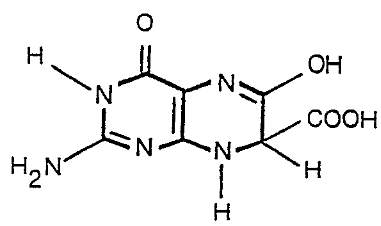

das 7,8-Dihydro-isoxanthopterin

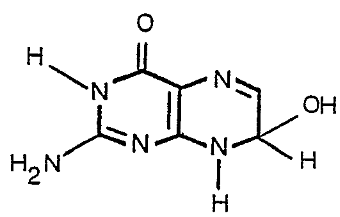

die 7,8-Dihydro-6-isoxanthopterincarbonsäure

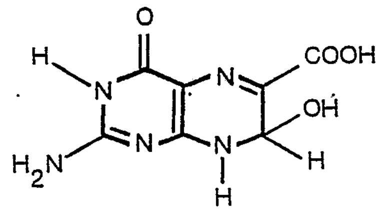

Die bisherigen Beispiele betrafen unkonjugierte Pteridine. Als Beispiel eines konjugierten Pteridins sei die Folsäure genannt:

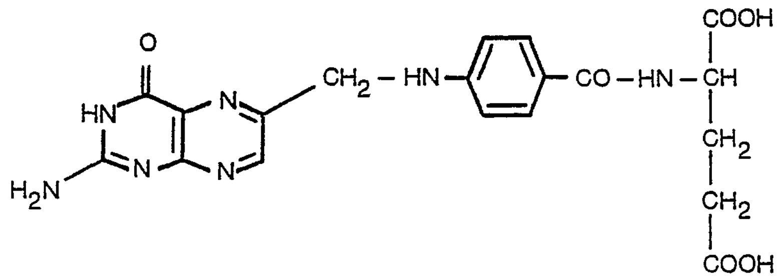

Aufgrund der eingangs erwähnten bekannten Bedeutung des Tetrahydrobiopterins als Cofaktor von fundamentalen Stoffwechselreaktionen war nicht zu erwarten, daß andere Pteridine eine viel deutlichere Aussage über Tumorwachstum geben könnten. Tatsächlich hat sich aber im Tierversuch herausgestellt, daß z.B. im Harn von Ehrlich-Ascites-Tumor tragenden oder virusinfizierten Mäusen eine signifikant erhöhte Ausscheidung von 7,8-Dihydro-6-hydroxylumazin, einem 7,8-Dihydropteridin auftritt, die sehr früh nachzuweisen ist und sehr gut mit dem Tumorwachstum oder mit der Schwere des Infektes der Modelltiere korreliert. Auch beim Menschen wurde festgestellt, daß das (vermehrte) Auftreten speziell von Neopterin in Körperflüssigkeiten ein sicherer Hinweis von malignem Wachstum ist. Völlig überraschend war der Befund, daß 7,8-Dihydropteridine auch bei Viruserkrankungen signifikant in Körperflüssigkeiten erhöht sind und daher ihre Bestimmung für die Virusdiagnostik herangezogen werden kann.

Das erfindungsgemäße Verfahren kann auf die speziellen Gegebenheiten beim Menschen dadurch vorteilhaft angepaßt werden, daß in der Körperflüssigkeit, vorzugsweise im Harn, Neopterin (6-Erythro-1,2,3 trihydroxypropyl-pterin) bzw. seine Reduktions- oder Oxidationsprodukte oder substituierten Produkte qualitativ nachgewiesen und/oder halbquantitativ bzw. quantitativ bestimmt werden.

Das erfindungsgemäß nachzuweisende Neopterin kann in folgender Weise vorliegen:

als D-Neopterin

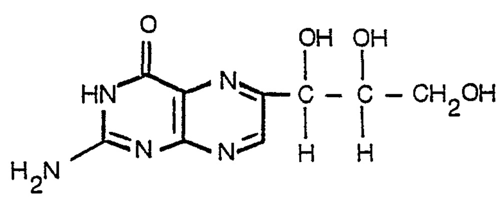

als L-Neopterin

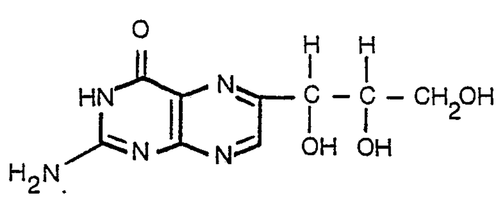

bzw. als Racemat des Neopterins.

Das erfindungsgemäß nachzuweisende Dihydropteridin kann auf unterschiedliche Art substituiert sein, beispielsweise durch Gruppen wie OH, $NH_2$, $CH_3$, $CH_2OH$, COOH, CHO, Alkohole, Phosphat oder Zucker. Demnach besteht eine spezielle Ausgestaltung des erfindungsgemäßen Verfahrens darin, daß im Harn oder Serum glykosidisch oder phosphatartig gebundenes Neopterin bzw. seine Reduktions- oder Oxidationsprodukte qualitativ nachgewiesen und/oder halbquantitativ bzw. quantitativ bestimmt werden.

Das Dihydropteridin, das Neopterin bzw. seine Reduktions- oder Oxidationsprodukte oder substituierten Produkte können in verschiedenen Körperflüssigkeiten, z.B. Blut, Serum, Harn, Speichel u.a.

nachgewiesen werden. Das erfindungsgemäße Verfahren setzt dabei nicht unbedingt voraus, daß dem Organismus, dem die zu untersuchende Körperflüssigkeit entnommen wird, vorher ein Pteridinderivat bzw. ein in den Pteridinstoffwechsel eingreifendes Präparat verabreicht wird, vielmehr ist es für das erfindungsgemäße Verfahren charakteristisch, daß es auch ohne vorherige Verabreichung eines Pteridinderivats bzw. ein in den Pteridinstoffwechsel eingreifendes Präparat durchführbar ist. Demnach werden also durch das erfindungsgemäße Verfahren vorzugsweise natürliche, im menschlichen oder tierischen Organismus selbst produzierte Pteridine bzw. Pteridinderivate bestimmt.

Der qualitative Nachweis eines 7,8-Dihydropteridins oder des Neopterins bzw. seiner Oxidationsprodukte, Reduktionsprodukte oder substituierten Produkte genügt, wenn der betreffende gesunde Organismus, d.h. ohne Vorhandensein eines malignen Tumors oder einer viralen Erkrankung in die zu untersuchende Körperflüssigkeit kein 7,8-Dihydropteridin abgibt. Andernfalls ist eine quantitative bzw. halbquantitative Bestimmung des 7,8-Dihydropteridins oder des Neopterins bzw. seiner Oxidationsprodukte, Reduktionsprodukte oder substituierten Produkte nötig, wobei diese Werte mit Normalwerten (von Gesunden) je nach der speziellen Untersuchungsmethode der Körperflüssigkeit zu vergleichen sind. Zum Nachweis oder zur quantitativen Bestimmung der Pteridinderivate oder des Neopterins einschließlich der oxidierten, hydrierten oder substituierten Produkte eignen sich übliche Analysenverfahren wie:

— Niedervolt — oder Hochspannungselektrophorese auf Trägern oder trägerfrei kombiniert mit in-situ-Fluorometrie bzw. Remissionsmessung im UV- bzw. sichtbaren Bereich nach Anfärbung;
— Dünnschichtchromatographie kombiniert mit in-situ-Fluorometrie oder Remissionsmessung im UV- bzw. sichtbaren Bereich nach Anfärbung;
— Papierchromatographie kombiniert mit in-situ-Fluorometrie oder Remissionsmessung im UV- bzw. sichtbaren Bereich nach Anfärbung;
— Gaschromatographie;
— Gaschromatographie kombiniert mit Massenspektrometrie;
— Felddesorptionsmassenspektrometrie;
— Hochdruckflüssigkeitschromatographie und Fluoreszenzdetektion bzw. Detektion im UV- bzw. sichtbaren Bereich nach chemischer Reaktion bzw. elektrochemische Detektion;
— Hochdruckflüssigkeitschromatographie und Massenspektrometrie;
— Flüssigchromatographie an Ionenaustauschern;
— Photometrie;
— Fluorometrie;
— Spektroskopie (UV-, IR-, kernmagnetische Resonanz, Massenspektrometrie);
— Immunologische Methoden (Radio-Immuno-Assay (RIA), Enzyme-Linked-Immuno-Sorbent-Assay (ELISA), Enzym-Immuno-Assay (EMIT), Immunelektrophorese u.a.);
— Isotopenverdünnung;
— Enzymtest, z.B. mittels Xanthinoxidase;
— Farbtest;
— Biologischer Text (Crithidia-Wachstumtest u.a.);
— iso-elektrische Fokussierung,
— kinetische Nephelometrie.

Für die Praxis bevorzugt, da einerseits sehr spezifisch, andererseits mit relativ einfachen Mittels im Routinebetrieb einsetzbar, sind die immunologischen Methoden, wie RIA, ELISA und EMIT. Hierfür ist es erforderlich, Antikörper gegen die zu bestimmenden Pteridine, beispielsweise Xanthopterin oder Neopterin zu gewinnen. Diese Antikörper werden auf an sich bekanntem Weg durch Immunisierung von Versuchstieren, vorzugsweise Kaninchen, mit geeigneten Pteridin-Konjugaten gewonnen. Die Kupplung der Pteridine an die immunogenen Träger, z.B. Serumalbumine, erfolgt nach an sich bekannten Methoden. Die erforderliche Spezifität der Antikörper für das jeweils zu bestimmende Pteridin wird durch die Auswahl der zur Kupplung eingesetzten Pteridinderivate erhalten.

Antikörper für allgemein in 6-Stellung durch eine Seitenkette substituierte Pterine werden erhalten, indem die Kupplung des Haptens an den immunogenen Träger über die 6-Stellung erfolgt.

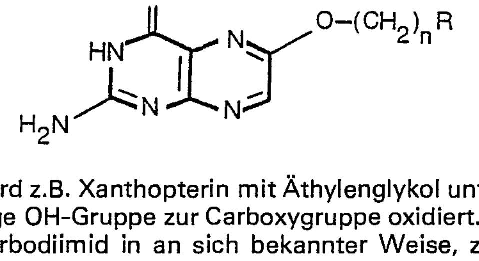

R = OH  n = 2,3, . . .
R = COOH  n = 1,2, . . .

Dabei wird z.B. Xanthopterin mit Äthylenglykol unter saurer Katalyse umgesetzt und anschließend die endständige OH-Gruppe zur Carboxygruppe oxidiert. Dieses reaktionsfähige Haptenderivat wird mittels einem Carbodiimid in an sich bekannter Weise, z.B. an Rinderserumalbumin, konjugiert.

Bei einem Antikörper, der spezifisch für Neopterin ist, muß die Trihydroxypropylseitenkette des Neopterins als Determinante bei der Kupplung des Haptens an den immunogenen Träger erhalten bleiben. Daher wird als einfachste Reaktion die Alkylierung, die vorwiegend in 3-Stellung des Moleküls

stattfindet, mit einem Alkylhalogenid durchgeführt, das zusätzlich eine, evtl. geschützte, weitere reaktionsfähige Gruppe besitzt.

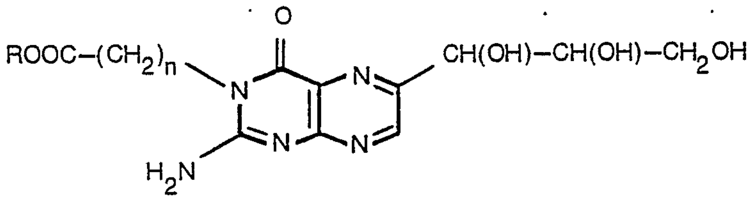

R = Me

R = H

n ≥ 3

Z.B. führt die Umsetzung von Neopterin mit ω-Bromvaleriansäuremethylester zum Neopteryl-ω-valeriansäuremethylester, der nach saurer Hydrolyse der Methylesterschutzgruppe mittels der Carbodiimidmethode an z.B. Rinderserumalbumin konjugiert wird.

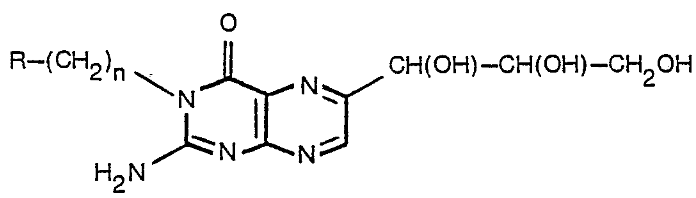

R = CN

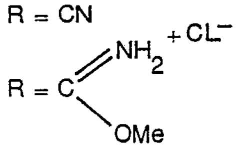

n ≥ 3

Entsprechend reagiert z.B. ω-Bromvaleronitril mit Neopterin zum 3-(4-Cyanobutyl-)neopterin, das durch Umsetzung in absolutem Methanol mit Chlorwasserstoff den entsprechenden Imidoester ergibt, der direkt mit Rinderserumalbumin umgesetzt werden kann.

Neopterin steht hierbei je nach dem gewünschten Antikörper für D-erythro-Neopterin, L-erythro-Neopterin, L-threo-Neopterin oder D-threo-Neopterin.

Spezifische Xanthopterin-Antikörper werden erhalten, indem die Kupplung des Haptens an den immunogenen Träger über die Position 3 erfolgt. Um Reaktionen an den sonst begünstigten Positionen 5 bzw. 6 auszuschließen, ist es im allgemeinen erforderlich, diese vorübergehend zu schützen, z.B. durch Methylierung des Sauerstoffs in 6-Stellung.

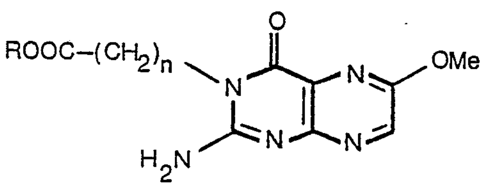

R = Me

R = H

n ≥ 3

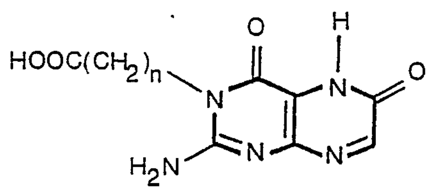

Die Alkylierung des Stickstoffatoms in 3-Stellung mit ω-Bromvaleriansäuremethylester wird durch vorherige Umsetzung mit Hexamethyldisilazan zum O-4-Trimethylsilylderivat erleichtert; andernfalls wird auch das O-4-Alkylderivat gebildet. Durch Erwärmen in Säure wird die 6-Methoxy- und die Methylesterschutzgruppe wieder entfernt.

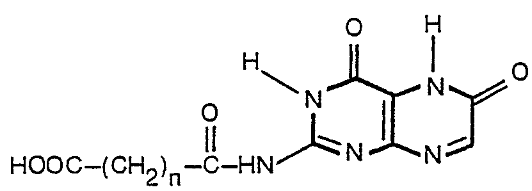

n = 2,3, . . .

Eine weitere Möglichkeit besteht in der Verknüpfung des Xanthopterins mit dem immunogenen Träger über die 2-Aminogruppe. Umsetzung mit Bernsteinsäureanhydrid in der Wärme ergibt das Hemisuccinat des Xanthopterins, das in üblicher Weise mittels Carbodiimid z.B. an Rinderserumalbumin konjugiert wird.

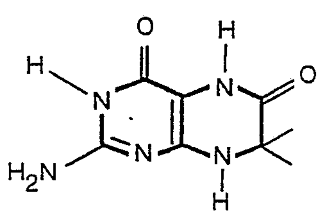

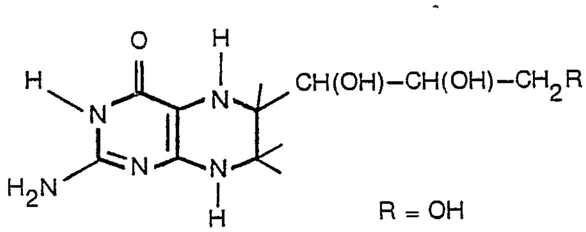

R = OH

Für die Bestimmung reduzierter Formen der Pteridine können ebenfalls Antikörper erzeugt werden. Dazu werden entweder die zuvor beschriebenen Konjugate einer Reduktion mit in der Pteridinchemie üblichen Verfahren unterzogen oder die hier beschriebenen reaktionsfähigen Pteridinderivate werden vor der Kupplung an immunogene Träger im gewünschten Maße hydriert. Dabei ist allerdings zu beachten, daß die reduzierten Pteridine, vor allem die 5,6,7,8-Tetrahydroderivate sauerstoffempfindlich und noch lichtempfindlicher als die oxidierten Formen sind und wegen ihrer Sauerstoffempfindlichkeit im allgemeinen etwas schwieriger bestimmbar sind.

Diese reaktionsfähigen Pteridinderivate werden außer zu Verknüpfungen mit dem immunogenen Träger auch zur Herstellung der bei immunologischen Verfahren notwendigen Tracer (radioaktiv oder durch Enzyme markiert) verwendet.

Dabei wird z.B. das 3-(4-Carboxybutyl-)D-erythro-neopterin mit 3-Radiojodtyramin nach der Carbodiimidmethode umgesetzt, wobei i.a. $^{125}$J verwendet wird. Dieser Tracer ist für die radioimmunologische Bestimmung von Neopterin besonders geeignet.

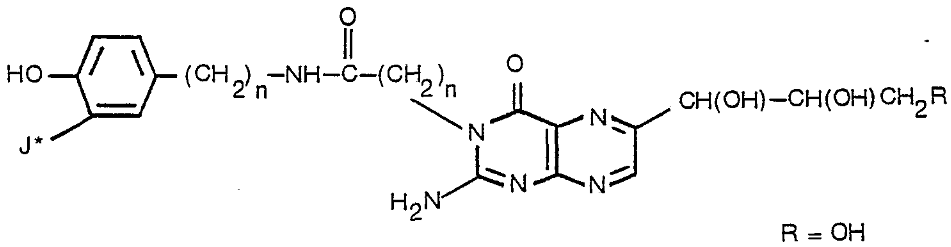

R = OH

Bei der Herstellung von Enzymkonjugaten wird ähnlich verfahren. Die Umsetzung von z.B. Peroxidase (z.B. Meerrettich) und z.B. 3-(4-Carboxybutyl-)D-erythro-neopterin nach der Carbodiimidmethode ergibt ein Enzymkonjugat, das zur enzymimmunologischen Bestimmung von Neopterin verwendet wird.

Nachstehend wird das erfindungsgemäße Verfahren anhand von Beispielen näher erläutert:

## Beispiel 1

Zum Nachweis eines malignen Wachstums und/oder viralen Erkrankungen beim Menschen eignet sich die quantitative Bestimmung des 7,8-Dihydroxanthopterin im Harn durch dünnschichtchromatographische Trennung des nativen Harnes. Nach Trocknung der Dünnschichtplatte erfolgt eine quantitative fluorometrische Direktauswertung 5 $\mu$l Humanharn werden portionsweise unter Zwischentrocknung mit einem Warmluftgebläse auf eine Cellulose-Dünnschichtplatte aufgetragen. In einer Dünnschichtkammer läuft das Laufmittel n-Butanol/Eisessig/Wasser (20/3/7) 12 cm weit. Nach Trocknung mit einem Warmluftgebläse wird die Dünnschichtplatte mit einem Chromatogrammspektralfluorometer gescannt und das Fluroeszenzsignal über einen Millivoltschreiber aufgezeichnet. Die Anregungswellenlänge beträgt hierbei 378 nm, die Meßwellenlänge 486 nm. Gemessen wird hierbei die Fluoreszenz eines Oxidationsproduktes, nämlich des Xanthopterins. Die Fläche der Bande des fluoreszierenden Fleckes bei Rf = 27 ist proportional dem im Harn ausgeschiedenen 7,8-Dihydroxanthopterin.

## Beispiel 2

Zum Nachweis des Ehrlich-Ascites-Tumor und/oder viraler Infekte bei der Maus eignet sich z.B. die quantitative Bestimmung des 7,8-Dihydro-6-hydroxylumazins im Harn durch hochspannungselektrophoretische Trennung des nativen Harnes und anschließender fluorometrischer Direktauswertung:

10 $\mu$l Mausharn werden auf einem mit Michaelispuffer (pH = 8,6 Ionenstärke = 0,75) getränkten Papierbogen strichförmig auf 3 cm Länge aufgetragen. Der Lauf erfolgt in einer kommerziellen Hochspannungselektrophoresekammer bei 2700 Volt und 45 Minuten Dauer. Nach Lufttrocknung wird der Elektrophoresebogen 16 cm weit in einem Chromatogrammfluoreszenzspektralphotometer gescannt, wobei das Fluoreszenzsignal mit einem Millivoltschreiber aufgezeichnet wird. Die Anregungswellenlänge beträgt hierbei 372 nm, die Meßwellenlänge 456 nm. Gemessen wird hierbei die Fluoreszenz eines Oxidationsproduktes des 7,8-Dihydro-6-hydroxylumazins, nämlich des 6-Hydroxylumazins. Die Fläche der Bande bei der relativen elektrophoretischen Mobilität (bezogen auf 4-Hydroxyhippursäure = 100) hrM̄ = 55 ist proportional dem im Harn ausgeschiedenen 7,8-Dihydro-6-hydroxylumazin.

## Beispiel 3

Ein weiteres mögliches Analysenverfahren zur Bestimmung des 7,8-Dihydro-6-hydroxylumazins besteht in Dünnschichtchromatographie auf Cellulose mit dem Laufmittel n-Butanol/Eisessig/Wasser (20/3/7). Der hRf-Wert des 7,8-Dihydro-6-hydroxylumazins liegt in diesem System bei 25. Auch hier erfolgt die quantitative Bestimmung des 7,8-Dihydro-6-hydroxylumazins durch Direkt-Fluorometrie bei 372 nm Anregung und 456 nm Messung mit einem Chromatogrammspektralfluorometer. Regt man bei 356 nm an und mißt man bei 440 nm, so kann man durch Direkt-Fluorometrie beim hRf-Wert 37 Neopterin quantitativ erfassen.

Beispiel 4

7,8-Dihydro-6-hydroxylumazin und sein Oxidationsprodukt 6-Hydrolumazin werden simultan quantitativ flüssigchromatographisch bestimmt. Hierbei wird 1 ml vorgereinigte Probelösung auf eine Niederdrucksäule von 82 cm Länge und 6 mm innerem Durchmesser, gefüllt mit reversed-phase-Material Bondapak $C_{18}$, Teilchengröße 37—75 $\mu$m, aufgegeben. Anschließend wird isokratisch mit einer 25 mM Ammoniumacetatlösung, pH 4,5, bei einem Durchfluß von 100 ml/Stunde eluiert. 7,8-Dihydro-6-hydroxylumazin hat ein Retentionsvolumen von 47—53 ml und wird durch seine UV-Absorption bei 254 nm gemessen.

6-Hydrolumazin wird von 54—60 ml eluiert und fluorometrisch bei 366 nm Anregung und Messung oberhalb 400 nm gemessen.

Beispiel 5

Zum Nachweis eines malignen Wachstums und/oder viralen Erkrankungen beim Menschen eignet sich z.B. die quantitative Bestimmung des Neopterins im Harn durch hochdruckflüssigchromatographische (HPLC) Trennung des nativen Harnes und Fluoreszenzdetektion. Gegebenenfalls kann vor der chromatographischen Trennung eine Vortrennung mittels Vorsäule od. dgl. erfolgen.

5 $\mu$l Humanharn bzw. die äquivalente Menge nach der Vorreinigung werden in das HPLC-System eingegeben. Am HPLC-System wird isokratisch gefahren. Als Puffer wird Ammonacetat/Essigsäure (30 m mol/Liter, pH = 6,0) mit 5% Methanolzusatz verwendet. Benützt wird eine 30 cm — Säule mit reversed-phase-Material als Füllmittel. Die Durchflußgeschwindigkeit beträgt 2 ml/min.

Durch das HPLC-System erfolgt eine klare Abtrennung des Neopterins von anderen Substanzen, so daß das Neopterin mittels Fluoreszenzdetektor und angeschlossenem Schreiber quantitativ erfaßt werden kann. Die Anregungswellenlänge beträgt hierbei 380 nm, die Meßwellenlänge 450 nm.

Gleichzeitig mit der Bestimmung des Neopterins wird das im Harn ausgeschiedene Kreatinin quantitativ bestimmt, so daß die Angabe in ng Neopterin pro mg Kreatinin erfolgen kann. Der Bezug auf Kreatinin empfiehlt sich immer dann, wenn nicht der Gesamt-Tagesurin, sondern nur übliche Urinproben zur Verfügung stehen.

Ein weiteres mögliches Analysenverfahren zur Bestimmung des Neopterins besteht in Dünnschichtchromatographie auf Cellulose mit dem Laufmittel 5%ige Essigsäure. Der hRf-Wert des Neopterins liegt in diesem System bei 65. Die quantitative Bestimmung des Neopterins erfolgt durch Direktfluorometrie bei 364 nm Anregung und 456 nm Messung mit einem Chromatogrammspektralfluorometer.

Beispiel 6

6-(2-Carboxy-)äthoxypterin

Im Dunkeln oder bei gedämpftem Licht wird 1 g Xanthopterin unter Rühren in 60 ml absolutem Propandiol-(1,3) auf 70°C (Ölbad) erhitzt. Dann wird trockener Chlorwasserstoff für ca. 3 h eingeleitet. Beim Einengen wird das Hydrochlorid erhalten, das durch Erhitzen (15 min.) mit Wasser das 6-(3-Hydroxy-)propyloxypterin ergibt.

0,5 g dieses Produktes werden in 20 ml 0,1 N NaOH mit $KMnO_4$-Lösung behandelt. Der ausgefallene Niederschlag wird abgesaugt, das Filtrat liefert beim Ansäuern das 6-(2-Carboxy-)Äthoxypterin.

Beispiel 7

3-(4-Carboxybutyl)D-erythro-neopterin

48 mg D-erythro-Neopterin werden in 20 ml absol. DMF mit 50 mg wasserfreiem $K_2CO_3$ und 0,2 ml $\omega$-Bromvaleriansäuremethylester unter Feuchtigkeitsausschluß bei Raumtemperatur gerührt. Nach 18 h werden weitere 0,2 ml $\omega$-Bromvaleriansäuremethylester zugegeben und für weitere 50 h gerührt. Danach werden 5 ml 2 N HCL bis zur sauren Reaktion (pH ca. 1—2) zugesetzt; nach 5 h wird im H.Vak. eingeengt, der Rückstand mit Wasser verrieben, abgesaugt und getrocknet.

Dieses Produkt kann direkt für die Konjugation an Rinderserumalbumin verwendet werden. Die Arbeiten werden wie im Beispiel 7 und in den folgenden Beispielen 8—14 im Dunkeln oder bei gedämpftem Licht durchgeführt.

Beispiel 8

3-(4-Methoxyiminocarbonylbutyl-)D-erythro-neopterin

48 mg D-erythro-Neopterin werden in 10 ml absol. DMF mit 40 mg wasserfreiem $K_2CO_3$ und 0,4 ml $\omega$-Bromvaleronitril unter Feuchtigkeitsausschluß bei 40°C (Bad) gerührt. Nach 2 Tagen wird im H.Vak. einrotiert, der Rückstand in wenig methanol. $NH_3$ gelöst und an Kieselgel schichtchromatographisch getrennt. Die dem 3-(4-Cyanobutyl-)D-erythro-Neopterin entsprechende Zone wird eluiert und einrotiert. 30 mg dieses Produktes werden in 10 ml HCl-gesättigtem Methanol unter Kühlung suspendiert und unter weiterem Einleiten von trockenem HCl langsam auf 10°C erwärmt. Die Mischung bleibt dann 24 h stehen. Nach Zugabe von 20 ml absol. Diäthyläther wird das Produkt abgesaugt, gewaschen und getrocknet.

Beispiel 9

3-(4-Carboxybutyl-)xanthopterin

360 mg 6-Methoxypterin werden in 50 ml Hexamethyldisilazan unter Feuchtigkeitsausschluß am Rückfluß erhitzt (20 h). Danach wird im Vak. eingeengt. Der Rückstand wird in 40 ml absol. DMF mit 1 ml ω-Bromvaleriansäureäthylester bei 40°C für 30 h umgesetzt. Das Lösungsmittel wird i. H.Vak. abgezogen. Der Rückstand wird schichtchromatographisch an Kieselgel gereinigt; die dem 3-(4-Carbomethoxybutyl-)6-methoxypterin entsprechende Zone wird eluiert.

Zur Abspaltung der Schutzgruppe werden 100 mg dieses Produktes in 30 ml Eisessig mit 3 ml 6 N HCL 4 h auf 70°C erhitzt. Danach wird i.Vak. einrotiert, der Rückstand aus Äthanol/Wasser umkristallisiert.

Beispiel 10

Xanthopterin-$N^2$-hemisuccinat

200 mg Xanthopterin werden in 50 ml absolutem Pyridin mit 250 mg Bernsteinsäureanhydrid auf 70°C erwärmt. Nach 16 h wird auf Eis gegossen, der Niederschlag abgesaugt und mit Wasser gewaschen. Das Produkt kann direkt zum Konjugieren verwendet werden.

Beispiel 11

3-(Butyl-4-carbonsäure-[3'-$^{125}$jod-]tyramid)-D-erythro-neopterin

Ca. 1 m Ci 3-$^{125}$Jodtyramin werden mit 0,1 mg 3-(4-Carboxybutyl-)D-erythro-neopterin gemäß Beispiel 7 in 200 μl absol. DMF unter Kühlung mit 80 μg Dicyclohexylcarbodiimid in 50 μl absol. Dioxan versetzt. Nach Stehen über Nacht und Erwärmen auf Raumtemperatur wird durch Papierchromatographie (Laufmittel n-Propanol 1/3% wässr. $NH_3$ 1,v/v) das gewünschte $^{125}$J-Neopterinderivat abgetrennt und mit methanol. $NH_3$ eluiert.

Beispiel 12

$N^2$-[$^3$H]Acetylxanthopterin

0,1 mg Xanthopterin (0,5 μMol) werden im verschlossenen Reaktionsgefäß 6 h mit 5 m Ci [$^3$H]Essigsäureanhydrid in 200 μl absol. DMF auf 100°C erhitzt. Die Reaktionsmischung wird durch Papierchromatographie (Laufmittel n-Buthanol 2/5 M Essigsäure 1, v/v) gereinigt. Die markierte Verbindung wird mit methanol. $NH_3$ eluiert.

Beispiel 13

Xanthopterinhemisuccinat-β-D-Galactosidase-Konjugat

14 mg Xanthopterin-$N^2$-hemisuccinat gemäß Beispiel 10 und 9,2 mg Tri-n-butylamin werden in 500 μl absol. DMF im Eisbad gekühlt, nach Zugabe von 6,6 mg Chlorameisensäureisobutylester wird 30 min. gerührt. Danach werden 5 mg β-D-Galactosidase in 5 ml Wasser zugesetzt; die Reaktion wird durch Zugabe von 0,1 N NaOH bei pH 9 und unter 10°C gehalten. Nach 5 h Reaktionszeit wird über Nacht im Kühlschrank aufbewahrt. Das Enzymkonjugat kann durch Chromatographie an Sephadex G 25 gereinigt werden.

Beispiel 14

3-(4-Carboxybutyl-)D-erythro-neopterin-Peroxidase-Konjugat

17 mg 3-(4-Carboxybutyl-)D-erythro-neopterin gemäß Beispiel 7 werden nach der "Gemischten-Anhydrid-Methode" mit Chlorameisensäureisobutylester in absol. DMF umgesetzt. Die Konjugation an 20 mg Peroxidase (HRP) erfolgt in DMF/0,5% $NaHCO_3$ (1:5), 6 h, $<10$°C. Das Konjugat kann durch Chromatographie an Sephadex G 25 gereinigt werden.

Beispiel 15

Enzymimmuno-Assay

In einem kleinen Zentrifugenröhrchen (Eppendorf oder Sarstedt) werden 100 μl Serum mit 400 μl PBS-Puffer (0,05 M Phosphat pH 7,4; 0,1 M NaCl; 0,1% Rinderserumalbumin), 100 μl Xanthopterin-Antiserum (Kaninchen 1:1000) und 100 μl Xanthopterin-β-D-galactosidase-Konjugat (ca. 50 ng) 5 h bei Raumtemperatur inkubiert. Nach Zugabe von 100 μl 2. Antikörper (Ziege, Anti-Kaninchen) wird 3 h bei Raumtemperatur (oder über Nacht im Kühlschrank) inkubiert. Nach Zentrifugieren wird der Überstand abgesaugt, das Pellet in 1 ml PBS-Puffer resuspendiert.

Nach Wiederholung des Zentrifugierens und Absaugens wird 0,1 ml Enzymsubstrat (0,1 M Phosphat pH 7,0, 2,3 mM O-Nitrophenyl-β-D-galactopyranosid, 1 mM $MgCl_2$, 10 mM Mercaptoäthanol) zugesetzt und 2 h bei 23°C inkubiert. Nach für Standards und Proben gleicher Zeit, z.B. 2 h, werden 1,5 ml 0,2 M $Na_2CO_3$ zugegeben und die optische Dichte bei 420 nm bestimmt.

Beispiel 16

Radioimmunoassay

In kleinen Zentrifugenröhrchen (Eppendorf, Sarstedt, o.ä.) werden 20 μl Urin, 300 μl 0,1 M Phosphat PH 7,4, 100 μl Xanthopterin-Antiserum (Kaninchen 1:1500 mit Zusatz von 7 μg Kaninchen-γ-glo-

bulin) und 100 $\mu l$ 2-[$^3H$-Acetyl]xanthopterin (in der Zählausbeute des Gerätes und der spez. Aktivität des Tracers angepaßter Menge) 4 h bei Raumtemperatur inkubiert. Danach werden 100 $\mu l$ 2. Antikörper (Anti-Kaninchen-$\gamma$-globulin-Serum, Ziege, 1:25) zugesetzt und für weitere 1,5 h inkubiert. Nach Zugabe von 500 $\mu l$ Wasser wird zentrifugiert und der Überstand abgesaugt. Der Niederschlag wird in 1 ml Wasser resuspendiert, erneut zentrifugiert und der Überstand abgesaugt.

Das Präzipitat wird anschließend in 50 $\mu l$ Soluene TM 100 gelöst, mit insgesamt 10 ml Insta-Gel-Szintillationsflüssigkeit (beides Packard Instrument Co.) in ein Szintillationsfläschchen überführt und im Zählgerät (Packard) gemessen.

Eine nach den vorstehenden Beispielen und generell nach dem erfindungsgemäßen Verfahren durchgeführte Untersuchung beansprucht nur einen geringen Zeitaufwand. So kann eine Untersuchung etwa nach Beispiel 5 in sechs Minuten durchgeführt werden. Die Untersuchungen nach dem erfindungsgemäßen Verfahren können auch beliebig oft wiederholt werden und ermöglichen so auch eine Verlaufskontrolle der Viruserkrankung bzw. einer Therapie von malignen Tumoren.

Von dem in der Körperflüssigkeit befindlichen Pteridin bzw. von dem angewendeten Bestimmungsverfahren hängt es ab, ob das ursprüngliche Pteridin selbst erfaßt wird oder dessen z.B. durch Licht- und/oder Lufteinfluß entstehendes Abbauprodukt, Oxidationsprodukt bzw. Reduktionsprodukt.

**Patentansprüche**

1. Diagnostizierverfahren, insbesondere zur Früherkennung von malignen Tumoren und/oder viralen Erkrankungen im menschlichen oder tierischen Organismus, wobei eine dem Organismus entnommene Körperflüssigkeit auf den Gehalt bestimmter Pteridine untersucht wird, dadurch gekennzeichnet, daß in der Körperflüssigkeit 7,8-Dihydropteridin bzw. seine Oxidationsprodukte oder seine Reduktionsprodukte oder deren substituierte Produkte, ausgenommen Biopterin und seine Reduktionsprodukte, qualitativ nachgewiesen und/oder halbquantitativ bzw. quantitativ bestimmt werden.

2. Verfahren nach Anspruch 1, dadurch gekennzeichnet, daß im Harn oder Serum Neopterin (6-Erythro-1,2,3-trihydroxypropyl-pterin) bzw. seine Reduktions- oder Oxidationsprodukte oder deren substituierte Produkte qualitativ nachgewiesen und/oder halbquantitativ bzw. quantitativ bestimmt werden.

3. Verfahren nach Anspruch 2, dadurch gekennzeichnet, daß im Harn oder Serum glykosidisch oder phosphatartig gebundenes Neopterin bzw. seine Reduktions- oder Oxidationsprodukte oder deren substituierte Produkte qualitativ nachgewiesen und/oder halbquantitativ bzw. quantitativ bestimmt werden.

4. Verfahren nach Anspruch 2 oder 3, dadurch gekennzeichnet, daß D-Neopterin, L-Neopterin oder das Racemat des Neopterins qualitativ nachgewiesen und/oder halbquantitativ bzw. quantitativ bestimmt wird.

5. Verfahren nach Anspruch 1, dadurch gekennzeichnet, daß der Nachweis bzw. die Bestimmung des 7,8-Dihydropteridins bzw. seiner Oxidations- oder Reduktionsprodukte oder deren substituierte Produkte unter Bestrahlung und Messung der dabei entstehenden Fluoreszenzstrahlung erfolgt.

6. Verfahren nach Anspruch 1, dadurch gekennzeichnet, daß die quantitative Bestimmung des 7,8-Dihydropteridins bzw. seiner Oxidations- oder Reduktionsprodukte oder deren substituierte Produkte durch hochspannungselektrophoretische Trennung der Körperflüssigkeit und anschließender fluorometrischer Direktauswertung erfolgt.

7. Verfahren nach Anspruch 1, dadurch gekennzeichnet, daß die quantitative Bestimmung des 7,8-Dihydropteridins bzw. seiner Oxidations- oder Reduktionsprodukte oder deren substituierte Produkte durch Dünnschichtchromatographie und anschließender fluorometrischer Direktauswertung erfolgt.

8. Verfahren nach einem der Ansprüche 1 bis 4, dadurch gekennzeichnet, daß das 7,8-Dihydropteridin bzw. seine Oxidations- oder Reduktionsprodukte oder deren substituierte produkte quantitativ flüssigkeitschromatographisch bestimmt und fluorometrisch und/oder durch seine UV-Absorption gemessen werden.

9. Verfahren nach einem der Ansprüche 2 bis 4, dadurch gekennzeichnet, daß der Nachweis des Neopterins bzw. seiner Reduktions- oder Oxidationsprodukte oder deren substituierte Produkte mittels HPLC unter Beziehung auf Kreatinin fluorometrisch erfolgt.

10. Verfahren nach einem der Ansprüche 1 bis 4, dadurch gekennzeichnet, daß das 7,8-Dihydropteridin oder Neopterin bzw. seine Reduktions- oder Oxidationsprodukte oder deren substituierte Produkte immunologisch bestimmt werden.

11. Verfahren nach einem der Ansprüche 1 bis 4, dadurch gekennzeichnet, daß das 7,8-Dihydropteridin oder Neopterin bzw. seine Reduktions- oder Oxidationsprodukte oder deren substituierte Produkte immunologisch durch Radio-Immuno-Assay oder Enzym-Immuno-Assay bestimmt werden.

12. Verfahren nach einem der Ansprüche 1 bis 4, dadurch gekennzeichnet, daß das 7,8-Dihydropteridin oder Neopterin bzw. seine Reduktions- oder Oxidationsprodukte oder deren substituierte Produkte enzymatisch bestimmt werden.

13. Verfahren nach einem der Ansprüche 1 bis 12, dadurch gekennzeichnet, daß das 7,8-Dihydropteridin oder Neopterin bzw. seine Reduktions- oder Oxidationsprodukte oder deren substituierte Produkte in einer Körperflüssigkeit nachgewiesen bzw. bestimmt werden, die ohne vorherige Verabreichung eines Pteridinderivates bzw. eines in den Pteridinstoffwechsel eingreifenden Präparates dem Organismus entnommen wurde.

14. Verfahren nach Anspruch 11, dadurch gekennzeichnet, daß man Antikörper einsetzt, die durch Kupplung eines immunogenen Trägers an die entsprechenden Pteridin-Konjugate in 3- oder 6-Stellung gewonnen wurden.

15. Verfahren nach Anspruch 14, dadurch gekennzeichnet, daß man Tracer einsetzt, die durch Kupplung eines Enzyms oder einer radioaktiv markierten Gruppe an die betreffenden Pteridin-Konjugate in 3- oder 6-Stellung gewonnen wurden.

16. Diagnostisches Mittel zum immunologischen Nachweis von 7,8-Dihydropteridin bzw. seiner Reduktions- oder Oxidationsprodukte oder deren substituierte Produkte enthaltend einen Antikörper, der durch Kupplung eines immunogenen Trägers an die betreffenden Pteridin-Konjugate in 3- oder 6-Stellung gewonnen wurden, sowie eines Tracers, der durch Kupplung eines Enzyms oder einer radioaktiv markierten Gruppe an die betreffenden Pteridin-Konjugate in 3- oder 6-Stellung gewonnen wurde.

**Claims**

1. A diagnostic method, in particular for the early detection of malignant tumors and/or viral diseases in the human or animal organism, using a body fluid collected from the organism being examined for the content of specific pteridines, characterized in that 7,8-dihydropteridine or its oxidation products or its reduction products or their substituted products, with the exception of biopterin and its reduction products, is qualitatively detected and/or semi-quantitatively or quantitatively determined in the body fluid.

2. A method according to claim 1, characterized in that neopterin (6-erythro-1,2,3-trihydroxypropylpterin) or its reduction- or oxidation products or their substituted products are qualitatively detected and/or semi-quantitatively or quantitatively determined in the urine or serum.

3. A method according to claim 2, characterized in that neopterin bonded or its reduction- or oxidation products or their substituted products glycosidically or in a phosphatelike manner are qualitatively detected and/or semi-quantitatively or quantitatively determined in the urine or serum.

4. A method according to claim 2 or 3, characterized in that D-neopterin, L-neopterin or the racemate- of neopterin is qualitatively detected and/or semi-quantitatively or quantitatively determined.

5. A method according to claim 1, characterized in that the detection or determination of 7,8-dihydropteridine or its oxidation- or reduction products or their substituted products is effected by radiation and measurement of the resultant fluorescence radiation.

6. A method according to claim 1, characterized in that the quantitative determination of 7,8-dihydropteridine or its oxidation- or reduction products or their substituted products is effected by separation of the body fluid by high voltage electrophoresis and subsequent direct analysis by fluorometry.

7. A method according to claim 1, characterized in that the quantitative determination of 7,8-dihydropteridine or its oxidation- or reduction products or their substituted products is effected by thin layer chromatography and subsequent direct analysis by fluorometry.

8. A method according to one of claims 1 to 4, characterized in that 7,8-dihydropteridine or its oxidation- or reduction products or their substituted products are determined quantitatively by liquid chromatography and measured by fluorometry and/or by their U.V. absorption.

9. A method according to one of claims 2 to 4, characterized in that the detection of neopterin or its reduction- or oxidation products or their substituted products is effected fluorometrically by high pressure liquid chromatography with reference to creatinine.

10. A method according to one of claims 1 to 4, characterized in that 7,8-dihydropteridine or neopterin or its reduction- or oxidation products or their substituted products are immunologically determined.

11. A method according to one of claims 1 to 4, characterized in that 7,8-dihydropteridine or neopterin or its reduction- or oxidation products or their substituted products are immunologically determined by radio-immunoassay or enzyme immunoassay.

12. A method according to one of claims 1 to 4, characterized in that 7,8-dihydropteridine or neopterin or its reduction- or oxidation products or their substituted products are enzymatically determined.

13. A method according to one of claims 1 to 12, characterized in that 7,8-dihydropteridine or neopterin or its reduction- or oxidation products or their substituted products are detected or determined in a body fluid which was collected from the organism without prior administration of a pteridine derivative or a preparation interfering with the pteridine metabolism.

14. A method according to claim 11, characterized in that antibodies, obtained by coupling an immunogenic carrier to the respective pteridine conjugates in the 3- or 6-position, are used.

15. A method according to claim 14, characterized in that tracers are used which have been

obtained by coupling an enzyme or a radioactively labelled group to the respective pteridine conjugates in the 3- or 6-position.

16. Diagnostic preparation for the immunological detection of 7,8-dihydropteridine or its reduction- or oxidation products or their substituted products, comprising an antibody which has been obtained by coupling of an immunogenic carrier to the respective pteridine conjugates in the 3- or 6-position, as well as of a tracer which has been obtained by coupling of an enzyme or a radioactively labelled group to the respective pteridine conjugate in the 3- or 6-position.

**Revendications**

1. Méthode de diagnostic, en particulier de détection précoce de tumeurs malignes et/ou de maladies virales dans l'organisme humain ou animal, dans laquelle on examine une humeur prélevée sur l'organisme pour déterminer sa teneur en certaines ptéridines, caractérisée par le fait que l'on décèle qualitativement et/ou que l'on détermine semi-quantitativement ou quantitativement, dans l'humeur, la 7,8-dihydro-ptéridine ou ses produits d'oxydation ou ses produits de réduction ou ses produits substitués, à l'exception de la bioptérine et de ses produits de réduction.

2. Méthode selon la revendication 1, caractérisée par le fait que l'on décèle qualitativement et/ou que l'on détermine semi-quantitativement ou quantitativement, dans l'urine ou le sérum, la néoptérine (6-érythro-1,2,3-trihydroxypropyl-ptérine) ou ses produits de réduction ou d'oxydation ou ses produits substitués.

3. Méthode selon la revendication 2, caractérisée par le fait que l'on décèle qualitativement et/ou que l'on détermine semi-quantitativement ou quantitativement, dans l'urine ou le sérum, la néoptérine en combinaison glucosidique ou phosphatée ou ses produits de réduction ou d'oxydation ou ses produits substitués.

4. Méthode selon l'une des revendications 2 et 3, caractérisée par le fait que l'on décèle qualitativement et/ou que l'on détermine semi-quantitativement ou quantitativement la D-néoptérine, la L-néoptérine ou la néoptérine racémique.

5. Méthode selon la revendication 1, caractérisée par le fait que la détermination de la 7,8-dihydroptéridine ou de ses produits d'oxydation ou de réduction ou de ses produits substitués s'effectue sous irradiation et avec mesure du rayonnement de fluorescence ainsi apparu.

6. Méthode selon la revendication 1, caractérisée par le fait que la détermination quantitative de la 7,8-dihydroptéridine ou de ses produits d'oxydation ou de réduction ou de ses produits substitués s'effectue par séparation de l'humeur par électrophorèse à haute tension suivie d'une interprétation fluorométrique directe.

7. Méthode selon la revendication 1, caractérisée par le fait que la détermination quantitative de la 7,8-dihydroptéridine ou de ses produits d'oxydation ou de réduction ou de ses produits substitués s'effectue par chromatographie en couche mince suivie d'une interprétation fluorométrique directe.

8. Méthode selon l'une des revendications 1 à 4, caractérisée par le fait que l'on détermine quantitativement la 7,8-dihydroptéridine ou ses produits d'oxydation ou de réduction ou ses produits substitués par chromatographie liquide et qu'on la mesure par fluorométrie et/ou par son absorption d'ultraviolet.

9. Méthode selon l'une des revendications 2 à 4, caractérisée par le fait que le décèlement de la néoptérine ou de ses produits de réduction ou d'oxydation ou de ses produits substitués s'effectue par chromatographie liquide à haute pression avec référence à la créatinine par fluorométrie.

10. Méthode selon l'une des revendications 1 à 4, caractérisée par le fait que l'on détermine immunologiquement la 7,8-dihydroptéridine ou la néoptérine ou ses produits de réduction ou d'oxydation ou ses produits substitués.

11. Méthode selon l'une des revendications 1 à 4, caractérisée par le fait que l'on détermine immunologiquement la 7,8-dihydroptéridine ou la néoptérine ou ses produits de réduction ou d'oxydation ou ses produits substitués, par dosage radio-immunologique ou dosage enzymato-immunologique.

12. Méthode selon l'une des revendications 1 à 4, caractérisée par le fait que l'on détermine enzymatiquement la 7,8-dihydroptéridine ou la néoptérine ou ses produits de réduction ou d'oxydation ou ses produits substitués.

13. Méthode selon l'une des revendications 1 à 12, caractérisée par le fait que l'on décèle ou que l'on détermine la 7,8-dihydroptéridine ou la néoptérine ou ses produits de réduction ou d'oxydation ou ses produits substitués dans une humeur qui a été prélevée sur l'organisme sans administration préalable d'un dérivé de ptéridine ou d'une préparation intervenant dans le métabolisme de la ptéridine.

14. Méthode selon la revendication 11, caractérisée par le fait que l'on utilise des anticorps que l'on a obtenus en couplant un support immunogène avec les produits de conjugaison de ptéridine correspondants en position 3 ou 6.

15. Méthode selon la revendication 14, caractérisée par le fait que l'on utilise des traceurs que l'on a obtenus par couplage d'une enzyme ou d'un groupe marqué par radioactivité avec les produits de conjugaison de ptéridine considérés, en position 3 ou 6.

16. Agent diagnostique pour le décèlement immunologique de la 7,8-dihydroptéridine ou de ses produits de réduction ou d'oxydation ou de ses produits substitués, contenant un anticorps que l'on a obtenu en couplant un support immunogène avec les produits de conjugaison de ptéridine considérés, en position 3 ou 6, ainsi que d'un traceur que l'on a obtenu en couplant une enzyme ou un groupe marqué par radioactivité avec les produits de conjugaison de ptéridine considérés, en position 3 ou 6.